# EUROPÄISCHE PATENTSCHRIFT

(11) **EP 4 016 044 B1**
(45) Veröffentlichungstag und Bekanntmachung des Hinweises auf die Patenterteilung: **05.02.2025**
(21) Anmeldenummer: 20215089.2
(22) Anmeldetag: 17.12.2020
(51) Int. Cl.: G01N 1/30, G01N 33/483

(54) **VERBESSERTES VERFAHREN ZUR OPTISCHEN KLÄRUNG EINER GEWEBEPROBE ZUR LICHTMIKROSKOPISCHEN UNTERSUCHUNG**
IMPROVED METHOD FOR OPTICALLY CLARIFYING A TISSUE SAMPLE FOR MICROSCOPIC EXAMINATION
PROCÉDÉ AMÉLIORÉ DE CLARIFICATION OPTIQUE D'UN ÉCHANTILLON TISSULAIRE DESTINÉ À L'EXAMEN DE MICROSCOPIE OPTIQUE

(43) Veröffentlichungstag der Anmeldung: 22.06.2022
(73) Patentinhaber: MobiCron GmbH, 37083 Göttingen (DE)
(72) Erfinder: DIEKMANN, Stephan, 86911Dießen (DE); WOUTERS-BUNT, Fred, 37085 Göttingen (DE)
(74) Vertreter: Patentanwälte Olbricht Buchhold Keulertz

(56) Entgegenhaltungen:
- WO-A1-2017/093323
- MEIGGS THEODORE O. ET AL: "Extinction Coefficient and Recombination Rate of Benzyl Radicals. II Photolysis of Several Benzyl Phenylacetates in Methanol", JOURNAL OF THE AMERICAN CHEMICAL SOCIETY, vol. 94, no. 23, 15 November 1972 (1972-11-15), pages 7986 - 7991, XP055810476
- HANS-ULRICH DODT ET AL: "Ultramicroscopy: three-dimensional visualization of neuronal networks in the whole mouse brain", NATURE METHODS, vol. 4, no. 4, 25 March 2007 (2007-03-25), New York, pages 331 - 336, XP055590656, ISSN: 1548-7091, DOI: 10.1038/nmeth1036
- THRANE LARS ET AL: "Optical tissue clearing improves usability of optical coherence tomography (OCT) for high-throughput analysis of the internal structure and 3D morphology of small biological objects such as vertebrate embryos", PROGRESS IN BIOMEDICAL OPTICS AND IMAGING, SPIE - INTERNATIONAL SOCIETY FOR OPTICAL ENGINEERING, BELLINGHAM, WA, US, vol. 8953, 4 March 2014 (2014-03-04), pages 895305 - 895305, XP060034005, ISSN: 1605-7422, ISBN: 978-1-5106-0027-0, DOI: 10.1117/12.2037303

## Beschreibung

Die Erfindung betrifft ein Verfahren gemäß dem Oberbegriff von Anspruch 1, ein Kit zur Präparation von biologischen Gewebeproben gemäß Anspruch 10 und eine Verwendung gemäß Anspruch 12.

Transparente biologische Gewebeproben werden benötigt, um die Gewebeproben zum Beispiel mittels Lichtblattmikroskopie dreidimensional abbilden zu können. Um die Transparenz von biologischen Präparaten zu erreichen, sind insbesondere Häm-Gruppen des Blutfarbstoffs Hämoglobin und Lipide aus den biologischen Präparaten zu entfernen. Bei den "entwässernden" Verfahren wird das Gewebe mit verschiedenen Mischungen eines mit Wasser mischbaren organischen Lösungsmittels und Wasser behandelt. Die Behandlung wird mit einem aufsteigenden Anteil des organischen Lösungsmittels durchgeführt, um das Wasser vollständig aus dem Gewebe zu entfernen. Hier gibt es mehrere Möglichkeiten, wie z.B. Tetrahydrofuran, Methanol, Isopropanol, tert. Butanol und Ethanol. Für das pathologische Gewebeclearing ist Ethanol zurzeit das meist genutzte Entwässerungsmedium. Das Endresultat aller "entwässernden" Verfahren ist eine wasserfreie Probe.

Der letzte Schritt der verschiedenen Clearingverfahren ist das Angleichen des Brechungsindexes an den Brechungsindex des zu mikroskopierenden Gewebes. Der Brechungsindex von entwässertem Gewebe beträgt laut Spalteholz ("Über das Durchsichtigmachen von menschlichen und tierischen Präparaten", Verlag von S. Hirzel, 1911, Deutsches Reichs-Patent Nr. 229044) n = 1.547 für Knochen. Der Brechungsindex anderer Gewebe lässt sich anhand der von ihm empirisch ermittelten optimalen Mischungen auf ca. n = 1.551 abschätzen. Ein dermaßen hoher Brechungsindex erfordert den Einsatz aromatischer Verbindungen, die generell nicht oder nur geringfügig mit Wasser mischbar sind. Spalteholz nutzte für seine Untersuchungen Mischungen aus Wintergrünöl (Methylsalicylat) und Benzylbenzoat oder Isosafrol in einem den unterschiedlichen Geweben angepassten Mischungsverhältnis.

Eine Variante des Spalteholz'sche Verfahrens ist aus Dodt, Leischner, Schierloh, Jährling, Mauch, Deininger, Deussing, Eder, Zieglgänsberger, Becker "Ultramicroscopy: three-dimensional visualization of neuronal networks in the whole mouse brain". Nat Methods. 2007;4(4):331-6.) bekannt, bei dem "Murray's clear" (eine 2:1 Mischung aus Benzylbenzoat und Benzylalkohol mit einem Brechungsindex von n = 1.559) als Einbettungsmedium eingesetzt wird. Eine weitere Variante ist aus Becker, Jährling, Saghafi, Weiler, Dodt H "Chemical Clearing and Dehydration of GFP Expressing Mouse Brains" (2012) PLoS ONE 7(3): e33916. https://doi.org/10.1371/journal.pone.0033916 bekannt. Es werden Einbettungsmedien von Spalteholz sowie einige weitere aromatische Verbindungen getestet. Dibenzylether (DBE, Brechungsindex n = 1.562) stellte sich als am besten geeignete Verbindung heraus und hat sich zum de facto Goldstandard beim Gewebeclearing etabliert. Der Vorteil des DBE beruht auch darauf, dass es in der Nutzung einfach ist, weil die Lösung nicht angemischt und dann auf den Brechungsindex überprüft werden muss; es kann direkt als Reinstoff genutzt werden.

Aus WO 2017/093323 A1 ist die Verwendung von Ethylcinnamat (ECi) und verwandter Cinnamylester als ungiftige Alternativen zu den bis dahin gängigen Einbettungsmedien bekannt. Es zeigt eine geringere Toxizität als die anderen zur Gewebebehandlung eingesetzten aromatischen Verbindungen, wie Dibenzylether, Benzylalkohol oder Benzylbenzoat. Da es als reine Substanz den gewünschten Brechungsindex erreicht, entfällt ein Mischschritt und damit ein Arbeitsschritt und eine mögliche Fehlerquelle.

Der Auswahl an flüssigen Reinstoffen, die sich als Einbettungsmedien für entwässerte Proben eignen, beschränkt sich somit zurzeit auf die zwei Verbindungen Dibenzylether und Ethylcinnamat. Beide Substanzen haben aber jeweils Nachteile: Dibenzylether ist giftig. Ethylcinnamat hat zwar eine geringe Toxizität, sein Schmelzpunkt liegt aber bei 6- 9°C, so dass die mit Ethylcinnamat geklärten Proben nicht im Kühlschrank aufbewahrt werden können, da das Einbettungsmedium dort auskristallisiert. Ethylcinnamat hat außerdem einen vergleichsweise hohen Dampfdruck.

Der Erfindung liegt die Aufgabe zugrunde, ein Verfahren zur Herstellung transparenter Gewebeprobe eines biologischen Gewebes zur lichtmikroskopischen Untersuchung bereitzustellen, das die vorstehenden Nachteile überwindet und das insbesondere mit weniger Aufwand verbunden ist, als Mischungen als Einbettungsmedium herzustellen, Einbettungsmedien erhöhter Toxizität vermeidet und das gekühlte Lagern der entwässerten Gewebeproben erlaubt.

Die Aufgabe wird erfindungsgemäß gelöst durch ein Verfahren gemäß Patentanspruch 1. Die Aufgabe wird weiterhin gelöst durch ein Kit gemäß Patentanspruch 10 und die Verwendung gemäß Patentanspruch 12.

Weitere Ausführungsformen sind Gegenstand der Unteransprüche oder nachfolgend beschrieben.

Das erfindungsgemäße Verfahren zur Herstellung transparenter Gewebeproben eines biologischen Gewebes zur lichtmikroskopischen Untersuchung umfasst die Schritte:
a) Entwässern der Gewebeprobe mit einem entwässernden Lösungsmittel und
b) Klären der entwässerten Gewebeprobe durch Einlegen in ein flüssiges Einbettungsmedium mit einem zum Gewebe passenden Brechungsindex.

Das Einbettungsmedium enthält einen aromatischen Ester gemäß der Formel I oder Formel IV: 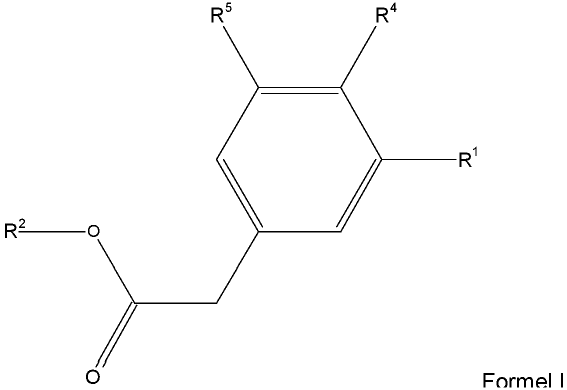 wobei
R¹ = -H, -CH₃ oder -CH₂-CH₃ ist,
R² ein Rest der Formel II oder der Formel III ist 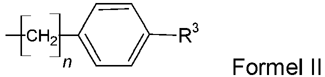 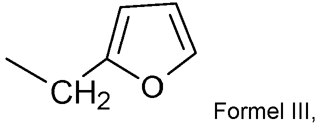
R³ = -H, -CH₃, -CH₂-CH₃ oder -OCH₃ ist
R⁴ = -H oder -CH₃ ist
R⁵ = -H oder -CH₃ ist und
n = 1 oder 2 ist;
   oder

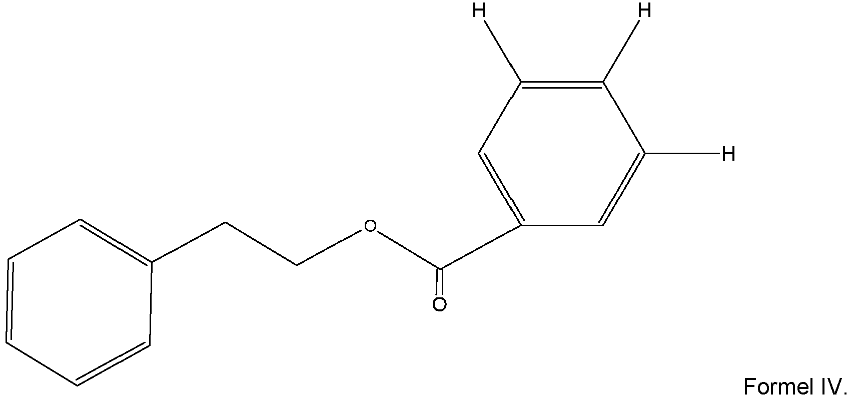

Bevorzugt ist der erfindungsgemäße Ester ein aromatischer Ester gemäß Formel I.

Das erfindungsgemäße Verfahren dient dazu, die optische Transparenz einer biologischen bzw. humanen Gewebeprobe für die Lichtmikroskopie zu erreichen. Das biologische Gewebe ist beispielweise humanes Gewebe oder tierisches Gewebe.

Der Klärungsschritt des Clearingverfahrens ist das Angleichen des Brechungsindexes an den Brechungsindex des zu mikroskopierenden Gewebes. Die Gewebeprobe wird hierzu in eine Brechungsindex-angleichende Lösung, das Einbettungsmedium, überführt. Das Einbettungsmedium muss mit dem Lösungsmittel des Entwässerungsschritts mischbar sein.

Das Einbettungsmedium enthält in einer Ausführungsform 10 bis 100 Vol.-% des aromatischen Esters und 0 bis 90 Vol.-% eines optisch geeigneten, inerten organischen Lösungsmittels mit einem Brechungsindex von etwa 1,3 bevorzugt von etwa 1,5. Beispielsweise enthält das Einbettungsmedium 90 bis 100 Vol.-% des aromatischen Esters gemäß Formel I.

Das Einbettungsmedium enthält in einer anderen Ausführungsform 10 bis 100 Vol.-% des aromatischen Esters und 0 bis 90 Vol.-% eines optisch geeigneten, inerten organischen Lösungsmittels mit einem Brechungsindex von etwa 2,0, bevorzugt von etwa 1,65. Beispielsweise enthält das Einbettungsmedium 90 bis 100 Vol.-% des aromatischen Esters gemäß Formel I.

Alle erfindungsgemäßen Prozentangaben erfolgen in Volumenprozent (Vol.-%).

In der bevorzugten Ausführungsform besteht das Einbettungsmedium aus einem aromatischen Ester gemäß Formel I oder der Formel IV, bevorzugt der Formel I, d.h. der aromatische Ester wird als Reinsubstanz eingesetzt. Als Reinsubstanz wird dabei der Ester in der technisch verfügbaren Reinheit verstanden.

Der aromatische Ester ist bevorzugt ein Phenylessigsäureester mit aromatischen Alkoholen. Der aromatische Ester ist besonders bevorzugt ausgewählt aus Benzyl-2-phenylacetat, 4-Methoxybenzyl-phenylacetat, Phenethylbenzoat oder 2-Furylmethyl-phenylacetat, besonders bevorzugt Benzyl-2-phenylacetat. Benzyl-2-phenylacetat (CAS Nr. 102-16-9) weist als technisches Produkt eine Reinheit von ca. 98,0 % auf. 4-Methoxybenzyl-phenylacetat (CAS Nr. 102-17-0) weist als technisches Produkt eine Reinheit von ca. 99,0 %auf. 2-Phenethylphenylacetat (CAS Nr. 102-20-5) weist als technisches Produkt eine Reinheit von ca. 99,0 % auf. 2-Furylmethyl-phenylacetat (CAS Nr. 36707-28-5) weist als technisches Produkt eine Reinheit von ca. 96,0 % auf. Phenethylbenzoat (CAS Nr. 94-47-3) weist als technisches Produkt eine Reinheit von ca. 98 % auf.

Der Entwässerungsschritt a) dient dazu, eine wasserfreie Gewebeprobe zu erhalten. Die Gewebeprobe wird in einer bevorzugten Ausführungsform in mehreren Durchgängen bevorzugt mit verschiedenen Dehydratisierungs-Zusammensetzungen mit einer absteigenden Wasserreihe behandelt, d.h. Mischungen mit einem aufsteigenden Anteil eines mit Wasser mischbaren organischen Lösungsmittels, um das Wasser vollständig aus dem Gewebe zu entfernen. Als entwässerndes Medium werden Alkohole, Ether oder Ketone eingesetzt. Geeignete entwässernde Lösungsmittel sind z.B. Ethanol, Methanol, Isopropanol, Tert. Butanol (IUPAC: 2-Methylpropan-2-ol), Tetrahydrofuran oder Aceton. Beim erfindungsgemäßen Verfahren umfasst der Entwässerungsschritt a) beispielsweise die Anwendung von Dehydratisierungs-Zusammensetzungen
- aus wässrigem Ethanol mit steigenden Konzentrationen von Ethanol, wobei die Ethanolkonzentrationen der Dehydratisierungs-Zusammensetzungen von 30% bis 100 Vol-% reichen oder
- aus wässrigem Tetrahydrofuran mit steigenden Konzentrationen von Tetrahydrofuran, wobei die Tetrahydrofurankonzentration der Dehydratisierungs-Zusammensetzungen von 30% bis 100 Vol-% reichen oder
- aus wässrigem Methanol mit steigenden Konzentrationen von Methanol, wobei die Methanolkonzentrationen der Dehydratisierungs-Zusammensetzungen von 30% bis 100 Vol-% reichen oder
- aus einer wässrigen Mischung eines anderen Alkohols, Ethers oder Keton, wobei die Lösungsmittelkonzentration der Dehydratisierungs-Zusammensetzungen von 30% bis 100 Vol-% reichen.

Das Entwässerungsmedium hat dabei folgende Eigenschaften: 1. es mischt sich vollständig mit Wasser, damit über eine aufsteigende Konzentrationsreihe dem Gewebe schrittweise das Wasser und das Fixationsmittel entzogen werden kann und 2. es mischt sich vollständig mit dem Einbettungsmedium, welches den Brechungsindex an den des entwässerten Gewebes angleicht.

In einer alternativen Ausführungsform erfolgt der Entwässerungsschritt a) mit 2,2-Dimethoxypropan (DMP), das durch eine chemische Reaktion mit dem im Gewebe enthaltenen Wasser dieses Wasser aus dem Gewebe entfernt.

In einer Ausführungsform des erfindungsgemäßen Verfahrens wird die Gewebeprobe, bevor sie in Schritt a) dehydriert und Schritt b) optisch geklärt wird,
- fixiert und/oder
- mit Formaldehyd fixiert und/oder
- gewaschen und/oder
- mit Wasser gewaschen und/oder
- in einer wässrigen alkalischen Lösung mit nicht-ionischem Detergens inkubiert. und/oder
- mit einer Detergenzienlösung delipidiert (Delipidierung) und/oder
- mit einem organischen Lösungsmittel delipidiert (Delipidierung) und/oder
- mit oxidierenden Reagenzien gebleicht und/oder
- mit Aminoalkoholen entfärbt.

In einer Ausführungsform des erfindungsgemäßen Verfahrens wird die Gewebeprobe, bevor sie in Schritt a) dehydriert und Schritt b) optisch geklärt wird, fixiert und das Fixierungsmittel ausgewählt aus
- vernetzenden Fixantien wie Formaldehyd, Glutaraldehyd, Acrolein, Carbodiimide, Diethylpyrocarbonat, Bisimodo-Ester oder Glyoxal oder Mischungen hiervon, und/oder
- koagulierenden Fixantien wie Alkoholen und anderen organischen Lösungsmitteln, Säuren, Kaliumdichromat, Bleinitrat, Kupfersulfat und Quecksilberchlorid und Mischungen hiervon.

Das erfindungsgemäße Verfahren wird bevorzugt nach Gewebevorbereitungsschritten und nach Elektrophorese-Schritten durchgeführt. Die im erfindungsgemäßen Verfahren behandelte Gewebeprobe ist somit bevorzugt bereits vorbehandelt. Die Vorbehandlung durch Elektrophorese erfolgt bevorzugt gemäß dem in DE 10 2016 123 458 B3 beschriebenen Verfahren zur elektrophoretischen Klärung. Für die Durchführung der elektrophoretischen Klärung wird Bezug genommen auf die Patentschrift DE 10 2016 123 458 B3, deren Inhalt hiermit in diese Anmeldung aufgenommen wird.

Die optisch geklärte Gewebeprobe wird im erfindungsgemäßen Verfahren bevorzugt in einem weiteren Schritt unter einem Mikroskop untersucht, um ein Bild der inneren Struktur der Probe zu erhalten, wobei das Mikroskop ein Lichtmikroskop, bevorzugt ein Lichtscheibenmikroskop, ein Konfokal-Mikroskop, ein Zwei-Photonen-Mikroskop oder ein optisches-Projektionstomographie-Mikroskop (OPT) ist.

Das bisher verwendete Einbettungsmedium Dibenzylether (DBE) weist einen Brechungsindex von n = 1.562 auf. Der zweite Reinstoff Ethylcinnamat besitzt einen Brechungsindex von n = 1.559.

Von den aromatischen Estern gemäß Formel I besitzt z. B. Phenylmethylbenzylacetat einen Brechungsindex von n = 1.555-1.558, 4-Methoxybenzyl-phenylacetat einen Brechungsindex von n = 1.559, 2-Phenethylphenylacetat einen Brechungsindex von n=1,551 und 2-Furylmethyl-phenylacetat einen Brechungsindex von n=1,548 (berechnet). Bei der Verwendung der aromatischen Ester gemäß Formel I als Einbettungsmedium für Gewebeclearing von entwässerten Gewebeproben konnte eine identische Durchsichtigkeit zu mit Methylsalicylat/Benzylbenzoat und mit Ethylcinnamat behandelten Gewebeproben erzielt werden. Neben Alkoxy-Substitutionen liefern insbesondere kurze Alkylsubstitutionen der Phenylessigsäureester ebenfalls Brechungsindices in diesem Bereich, der dem von Spalteholz ermittelten Brechungsindizes der Gewebe ähnelt.

Die Arbeiten von Spalteholz lassen bereits vermuten, dass sich die Brechungsindices unterschiedlicher Gewebe leicht voneinander unterscheiden. In seiner Originalveröffentlichung von 1911 wird folgende, an Hand des optimalen Mischungsverhältnisses von Methylsalicylat und BB empirisch bestimmten Reihe menschlicher Gewebe mit aufsteigendem Brechungsindex aufgeführt: junge Embryonen (5:1-3:1 je nach Gewicht) < erwachsene entkalkte Knochen (5:3) n = 1.547 < erwachsene Muskulatur ungefähr wie ältere Embryonen (2:1) < Gehirn und Rückenmark (1:1). Diese Variabilität hat direkte Folgen für die Durchsichtigkeit der unterschiedlichen Gewebe und deren mikroskopischen Untersuchung und insbesondere für quantitativ-spektroskopische Bestimmungen, welche mit einem Mikroskop durchgeführt werden können. Das erfindungsgemäße Verfahren bietet den Vorteil, dass verschiedene Reinstoffe als Einbettungsmedium eingesetzt werden, die je nach Gewebe ausgewählt werden können, um sich dem Brechungsindex des Gewebes anzunähern.

Gegenüber den bisher üblicherweise benutzten Einbettungsmedien Benzylbenzoat/Benzylalkohol-Mischungen (BABB) und Methylsalicylat-BenzylBenzoat-Mischungen besitzen die aromatischen Ester gemäß Formel I somit den Vorteil, dass sie, genau wie Dibenzylether, als reine Substanz eingesetzt werden können: Da bereits die reine Substanz den gewünschten Brechungsindex erreicht, muss der Brechungsindex nicht durch Anmischen des Einbettungsmediums eingestellt werden. Somit entfällt ein Mischschritt und damit ein überflüssig gewordener Arbeitsschritt und eine mögliche Fehlerquelle.

Die aromatischen Ester gemäß Formel I weisen zudem eine geringe Toxizität auf. Dieses ist bei der Benutzung als Einbettungsmedium vorteilhaft, da sich die Gewebeprobe im Mikroskop unmittelbar unterhalb der Atemwege des Benutzers befindet und dieser von der Probe aufsteigende Dämpfe einatmen kann. Die Toxizität von Phenylmethylbenzylacetat ist laut der Europäische Chemikalienagentur ECHA toxikologisch unbedenklich, anders als Dibenzylether oder Benzylbenzoat/Benzylalkohol-Mischungen.

Die Phenylessigsäureester gemäß Formel I sind zudem schwer flüchtig, da sie eine deutlich geringe Dampfspannung als die bekannten Einbettungsmedien haben, so dass weniger Dämpfe von der Gewebeprobe aufsteigen. Dieses reduziert zusätzlich die ohnehin geringe Toxizität der aromatischen Ester gemäß Formel I.

Ein Nachteil der bisher als Einbettungsmedien benutzten aromatischen Verbindungen ist ihre korrosive Wirkung. Es ist bekannt, dass die aromatischen Verbindungen das Objektiv und das Mikroskop angreifen, und dass schon die Dämpfe dieser flüchtigen Verbindungen ausreichen, um mit der Zeit die der Flüssigkeit zugewandten Oberflächen des Mikroskops zu korrodieren.

Durch die geringere Flüchtigkeit der aromatischen Ester gemäß Formel I bieten die erfindungsgemäßen Einbettungsmedien eine deutlich höhere Materialverträglichkeit, da wesentlich weniger Dämpfe aufsteigen, die zu einer Korrosion führen könnten. Zusammenfassend gilt: die geringe Flüchtigkeit der aromatischen Ester gemäß Formel I verringert die Geruchsbildung und die Atmungsreizung und -toxizität sowie die korrosive Einwirkung der Dämpfe auf das Mikroskop.

Phenylmethylbenzylacetat hat auch einen angenehmen Geruch, da es süß blumig, mit Noten von Jasmin und Schokolade riecht, was die Akzeptanz des Einbettungsmediums beim Laborpersonal erhöht. Beim Mikroskopieren wird der Benutzer also nicht durch unangenehme Gerüche beeinträchtigt, die vom Objekttisch aufsteigen, wie dies bei den herkömmlich genutzten Einbettungsmedien der Fall ist: Dibenzylether riecht unangenehm nach Plastik und Benzylalkohol riecht phenolisch.

Die Phenylessigsäurester gemäß Formel I und Formel IV erlauben zudem eine Aufbewahrung der geklärten Proben im Kühlschrank. Der Schmelzpunkt von beispielsweise Phenylmethylbenzylacetat liegt zwischen -13.54 °C bis -9 °C, d.h. die Verbindung ist somit Kühlschrank-tauglich und erlaubt sogar eine Aufbewahrung oder Transport auf Eis oder Kühlpackungen.

Das erfindungsgemäße Kit zur Präparation von biologischen Gewebeproben für die Lichtmikroskopie, enthält:
- ein entwässerndes Lösungsmittel zum Entwässern der Gewebeprobe und
- ein Einbettungsmedium zum Klären der entwässerten Gewebeprobe durch Einlegen in das Einbettungsmedium,
wobei
das Einbettungsmedium ein aromatischer Ester der Formel I oder Formel IV, wie vorstehend beschrieben, ist. Bevorzugt ist der aromatische Ester im erfindungsgemäßen Kit Benzyl-2-phenylacetat, 4-Methoxybenzyl-phenylacetat, Phenethylbenzoat oder 2-Furylmethyl-phenylacetat, bevorzugt Benzyl-2-phenylacetat. Der aromatische Ester wird bevorzugt in den vorstehend beschriebenen Konzentrationen und Reinheiten eingesetzt.

Erfindungsgemäß ist das entwässernde Lösungsmittel im Kit ausgewählt aus Ethanol, Methanol, Isopropanol, tert. Butanol, 2,2' Thiodiethanol, Trichloroethanol Tetrahydrofuran, oder Aceton.

Erfindungsgemäß erfolgt die Verwendung eines aromatischen Esters gemäß Formel I oder Formel IV mit 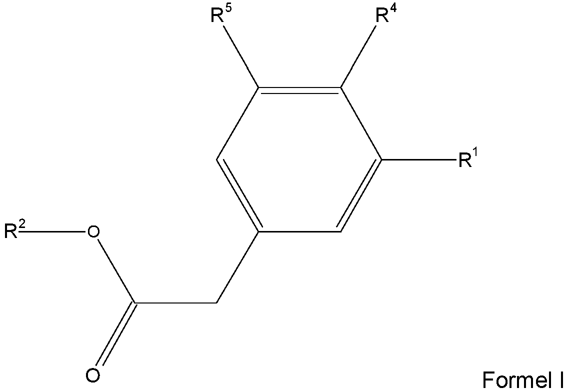 wobei
R¹ = -H, -CH₃ oder -CH₂-CH₃ ist,
R² ein Rest der Formel II oder der Formel III ist 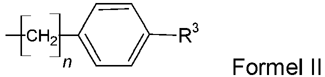 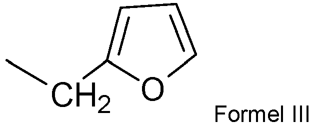
R³ = -H, -CH₃, -CH₂-CH₃ oder OCH₃ ist
R⁴ = -H oder -CH₃ ist
R⁵ = -H oder -CH₃ ist und
n = 1 oder 2 ist;
   oder 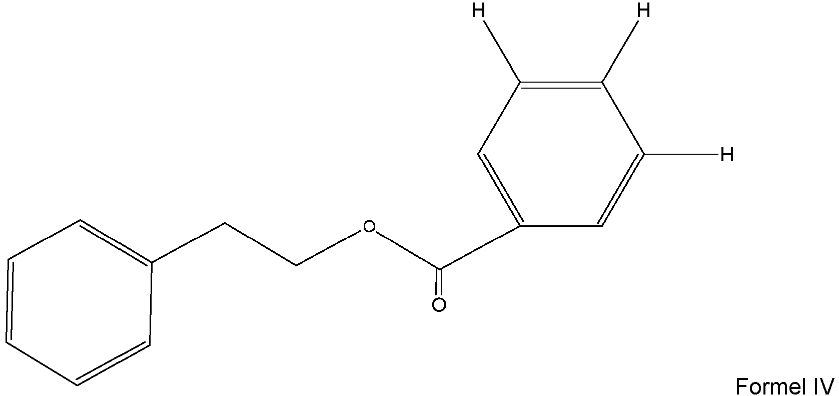
als Einbettungsmedium für die Präparation einer biologischen, insbesondere humanen, Gewebeprobe für die lichtmikroskopische Untersuchung.

Bevorzugt ist der aromatische Ester Benzyl-2-phenylacetat , 4-Methoxybenzyl-phenylacetat, Phenethylbenzoat oder 2-Furylmethyl-phenylacetat, besonders bevorzugt Benzyl-2-phenylacetat. Der aromatische Ester wird bevorzugt in den vorstehend beschriebenen Konzentrationen und Reinheiten verwendet.

Die Dampfspannungen verschiedener Einbettungsmedien sind in der Literatur bekannt.

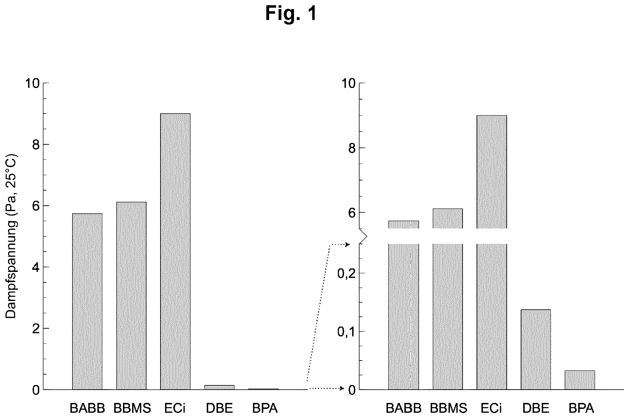

Fig. 1 zeigt die Dampfspannungen verschiedener Einbettungsmedien. Das erfindungsgemäße Phenylmethylbenzylacetat (Benzylphenylacetat (BPA)) besitzt eine deutlich geringere Dampfspannung als die nach heutigem Stand eingesetzten Einbettungsmedien. BPA ist von den verglichenen Einbettungsmedien das am geringsten flüchtige mit der niedrigsten Dampfspannung. Die Dampfspannung von BPA (bei 25 °C) ist ungefähr 275-fach geringer als die von Ethylcinnamat. Auch die beiden Einbettungsmedien BABB und BBMS besitzen deutlich (ung. 180-fach) höhere Dampfspannungen.

In einem vergrößerten Ausschnitt in Figur 1 ist erkennbar, dass BPA noch weniger (etwa 4-fach) flüchtig als das ebenfalls zwei aromatische Ringgruppen enthaltende DBE ist, welches auch weniger flüchtig ist als ECi aufgrund der deutlich geringeren Dampfspannung, mit geringerer Stärke als Methylsalicylat (auch in der Mischung mit BB) und ECi.

### Beispiele

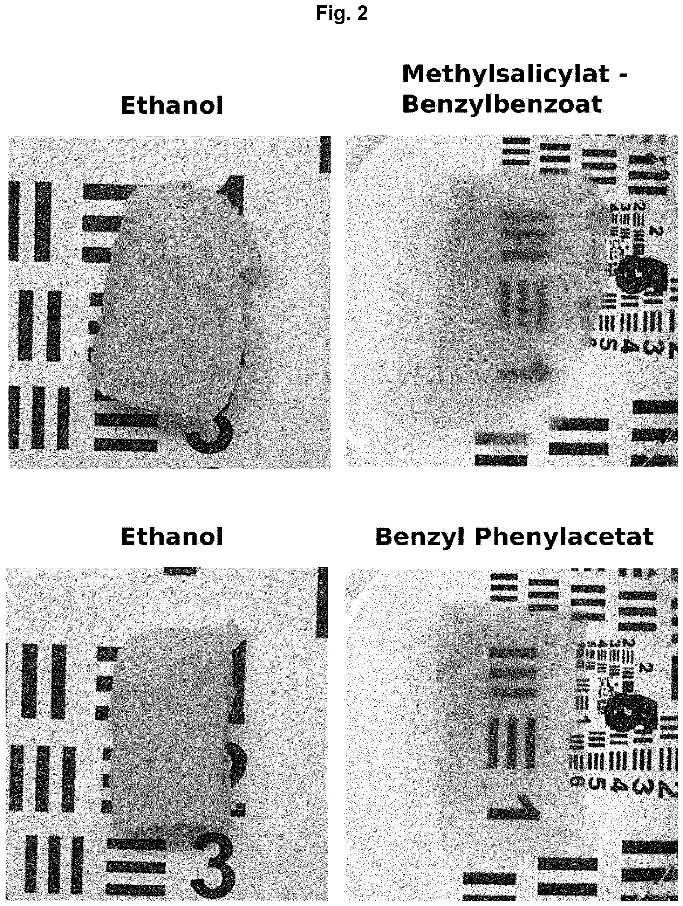

Eine Gewebeprobe (Schweinelunge) mit einem Volumen von etwa 0,25 ml (0,5x0,5x1 cm³) wurde mit Ethanol in mehreren Durchgängen entwässert. Im letzten Durchgang wurde hochreiner Ethanol verwendet und die entwässerte Gewebeprobe fotografiert. Die Ergebnisse sind in Figur 2, linke Seite dargestellt. Die Gewebeprobe wurde dann für 3 Stunden in 5 ml der jeweiligen Einbettungsmedien gegeben. Als Einbettungsmedien wurden ein Gemisch von Methylsalicylat mit Benzylbenzoat und das erfindungsgemäße Benzylphenylacetat verwendet. Das Resultat des Klärungsschritts wurde ebenfalls fotografiert und in Figur 2 rechte Seite dargestellt. Es zeigt sich, dass zwar bereits Methylsalicylat/Benzylbenzoat zu einer guten Durchsichtigkeit der Gewebeprobe führt. Mit dem erfindungsgemäßen Benzylphenylaectat konnte aber eine noch bessere Transparenz erzielt werden.

Die Erfindung ist nicht auf eine der vorbeschriebenen Ausführungsformen beschränkt, sondern in vielfältiger Weise abwandelbar.

## Patentansprüche

1. Verfahren zur Herstellung transparenter Gewebeprobe eines biologischen Gewebes zur lichtmikroskopischen Untersuchung, umfassend die Schritte:
a) Entwässern der Gewebeprobe mit einem entwässernden Lösungsmittel und
b) Klären der entwässerten Gewebeprobe durch Einlegen in ein Einbettungsmedium enthaltend einen aromatischen Ester gemäß Formel I oder Formel IV, mit 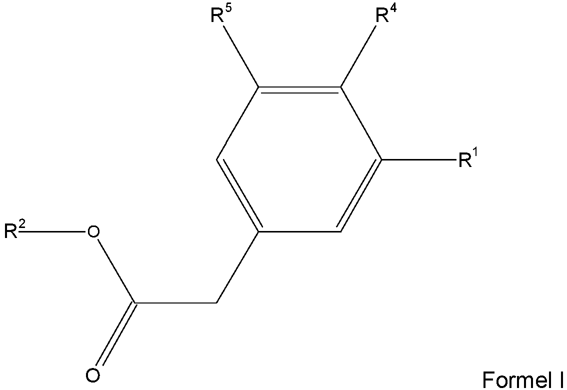 wobei
R¹ = -H, -CH₃ oder -CH₂-CH₃ ist,
R² ein Rest der Formel II oder der Formel III ist 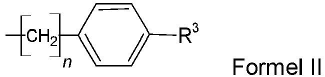 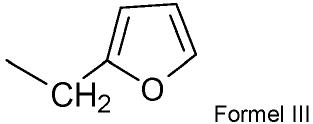
R³ = -H, -CH₃, -CH₂-CH₃ oder -OCH₃ ist
R⁴ = -H oder -CH₃ ist
R⁵ = -H oder -CH₃ ist und
n = 1 oder 2 ist;
oder

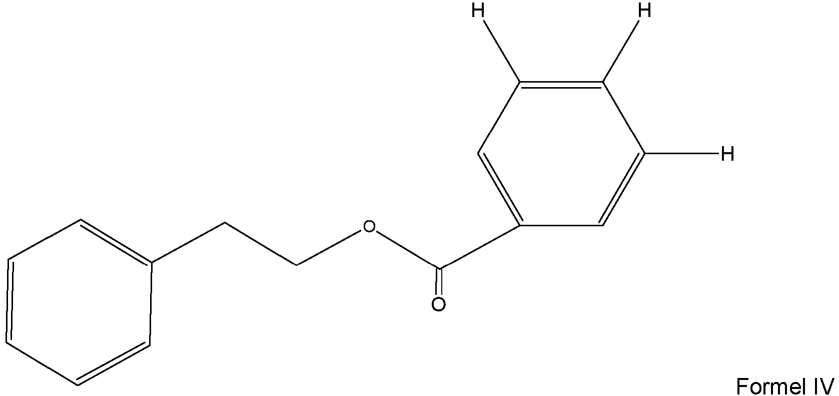

2. Verfahren gemäß Patentanspruch 1 **dadurch gekennzeichnet, dass** das Einbettungsmedium 10 bis 100 Vol.-% des aromatischen Esters enthält und 0 bis 90 Vol.-% eines optisch geeigneten, inerten organischen Lösungsmittels mit einem Brechungsindex von etwa 1.3, bevorzugt 1.5, enthält

3. Verfahren gemäß Patentanspruch 1 **dadurch gekennzeichnet, dass** das Einbettungsmedium 10 bis 100 Vol.-% des aromatischen Esters enthält und 0 bis 90 Vol.-% eines optisch geeigneten, inerten organischen Lösungsmittels mit einem Brechungsindex von etwa 2.0, bevorzugt 1.65, enthält.

4. Verfahren gemäß einem der vorhergehenden Ansprüche, **dadurch gekennzeichnet, dass** das Einbettungsmedium aus einem aromatischen Ester gemäß Formel I oder Formel IVbesteht.

5. Verfahren gemäß einem der vorhergehenden Ansprüche, **dadurch gekennzeichnet, dass** der aromatische Ester Benzyl-2-phenylacetat, 4-Methoxybenzyl-phenylacetat, Phenethylbenzoat oder 2-Furylmethyl-phenylacetat, bevorzugt Benzyl-2-phenylacetat, ist.

6. Verfahren gemäß einem der vorhergehenden Ansprüche, **dadurch gekennzeichnet, dass** der Entwässerungsschritt a) die Anwendung von Dehydratisierungs-Zusammensetzungen aus wässrigem Alkohol, Keton oder Ether umfasst.

7. Verfahren gemäß einem der vorhergehenden Ansprüche, **dadurch gekennzeichnet, dass** die Gewebeprobe, bevor sie in Schritt a) dehydriert und Schritt b) optisch geklärt wird,
• fixiert wird und/oder
• mit Formaldehyd fixiert wird und/oder
• gewaschen wird und/oder
• mit Wasser gewaschen wird und/oder
• in einer wässrigen alkalischen Lösung inkubiert wird und/oder
• mit einer Detergenzienlösung delipidiert wird und/oder
• mit einem organischen Lösungsmittel delipidiert wird und/oder
• mit oxidierenden Reagenzien gebleicht wird und/oder
• mit Aminoalkoholen entfärbt wird.

8. Verfahren gemäß einem der vorhergehenden Ansprüche, **dadurch gekennzeichnet, dass** die Gewebeprobe, bevor sie in Schritt a) dehydriert und Schritt b) optisch geklärt wird, fixiert wird und das Fixierungsmittel bevorzugt ausgewählt ist aus vernetzenden Fixantien wie Formaldehyd, Glutaraldehyd, Acrolein, Carbodiimide, Diethylpyrocarbonat, Bisimodo-Ester oder Glyoxal oder Mischungen hiervon, und/oder koagulierenden Fixantien wie Alkoholen und anderen organischen Lösungsmitteln, Säuren, Kaliumdichromat, Bleinitrat, Kupfersulfat und Quecksilberchlorid und ihre Mischungen.

9. Verfahren gemäß einem der vorhergehenden Ansprüche, **dadurch gekennzeichnet, dass** die optisch geklärte Gewebeprobe in einem weiteren Schritt unter einem Mikroskop untersucht wird, um ein Bild der inneren Struktur der Probe zu erhalten, wobei das Mikroskop ein Lichtmikroskop ist.

10. Kit zur Präparation von biologischen Gewebeproben für die Lichtmikroskopie, enthaltend:
- ein entwässerndes Lösungsmittel zum Entwässern der Gewebeprobe und
- ein Einbettungsmedium zum Klären der entwässerten Gewebeprobe durch Einlegen in das Einbettungsmedium,
wobei das entwässernde Lösungsmittel ausgewählt ist aus Ethanol, Tetrahydrofuran, Isopropanol, tert. Butanol, 2,2' Thiodiethanol, Trichloroethanol oder Aceton und in Form von Dehydratisierungs-Zusammensetzungen
aus wässrigem Lösungsmittel mit steigenden Konzentrationen von Lösungsmittel vorliegt und
das Einbettungsmedium ein aromatischer Ester der Formel I oder der Formel IV ist, mit 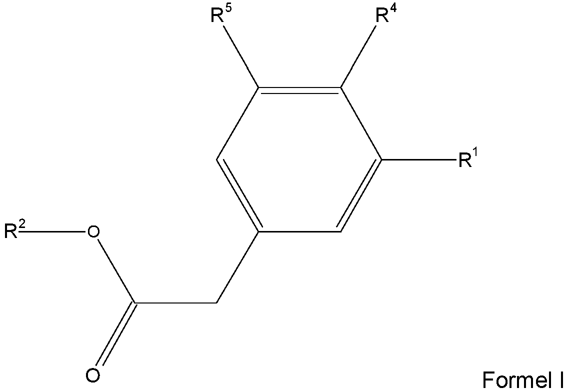 wobei
R¹ = -H, -CH₃ oder -CH₂-CH₃ ist,
R² ein Rest der Formel II oder der Formel III ist 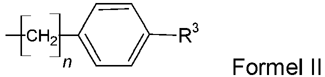 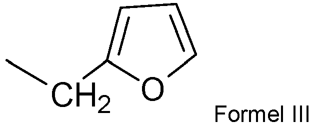
R³ = -H, -CH₃, -CH₂-CH₃ oder OCH₃ ist
R⁴ = -H oder -CH₃ ist
R⁵ = -H oder -CH₃ ist und
n = 1 oder 2 ist;
oder

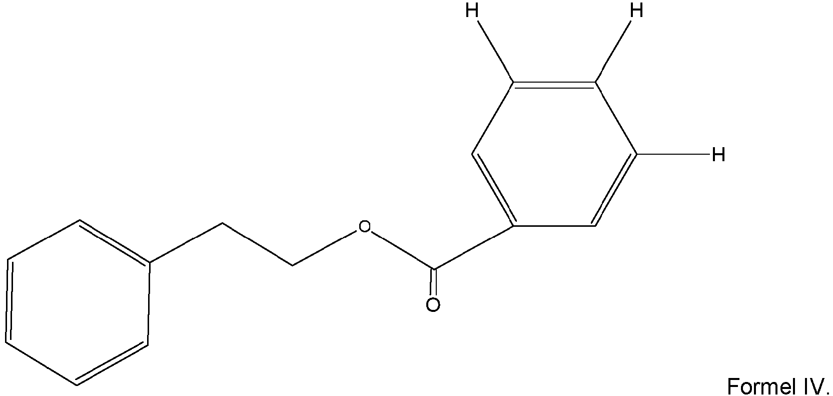

11. Kit gemäß Patentanspruch 10, **dadurch gekennzeichnet, dass** der aromatische Ester Benzyl-2-phenylacetat , 4-Methoxybenzyl-phenylacetat, Phenethylbenzoat oder 2-Furylmethyl-phenylacetat, bevorzugt Benzyl-2-phenylacetat, ist.

12. Verwendung eines aromatischen Esters gemäß Formel I oder gemäß Formel IV mit 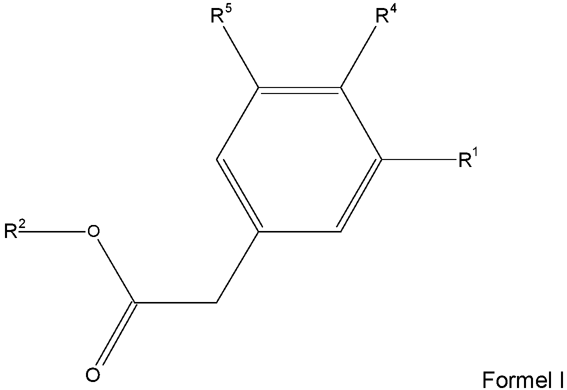 wobei
R¹ = -H, -CH₃ oder -CH₂-CH₃ ist,
R² ein Rest der Formel II oder der Formel III ist 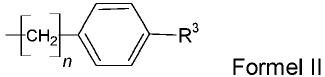 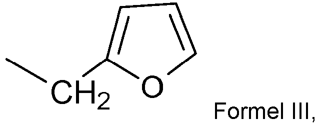
R³ = -H, -CH₃, -CH₂-CH₃ oder OCH₃ ist
R⁴ = -H oder -CH₃ ist
R⁵ = -H oder -CH₃ ist und
n = 1 oder 2 ist; oder 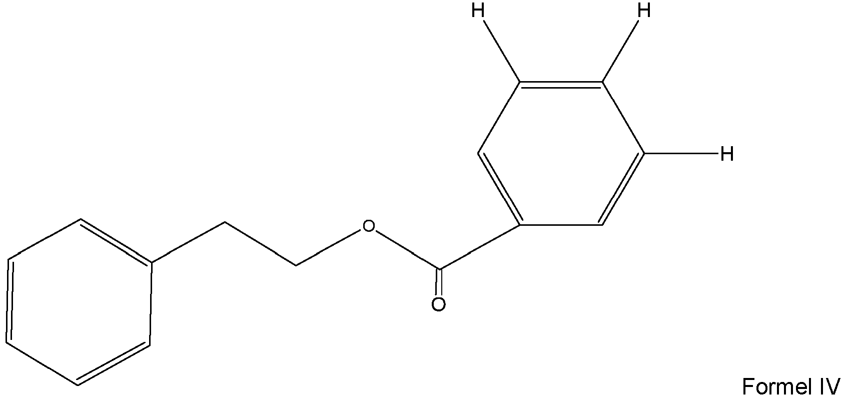
als Einbettungsmedium für die Präparation einer biologischen Gewebeprobe für die lichtmikroskopische Untersuchung.

## Claims

1. Method for preparing transparent tissue samples of a biological tissue for examination by light microscopy, comprising the steps of:
a) dehydrating the tissue sample with a dehydrating solvent and
b) clearing the dehydrated tissue sample by placing it in an embedding medium comprising an aromatic ester corresponding to formula I or formula IV, where 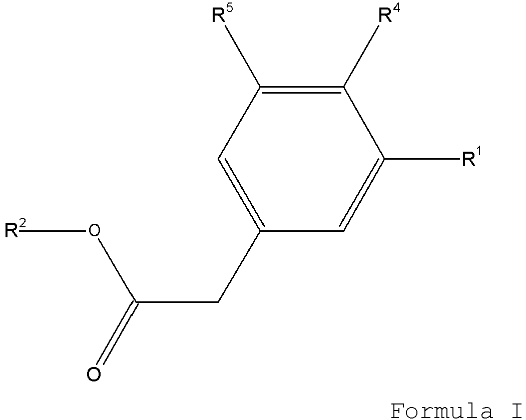 where
R¹ is -H, -CH₃ or -CH₂-CH₃,
R² is a radical of the formula II or formula III 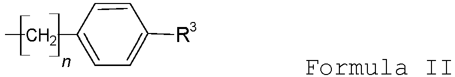 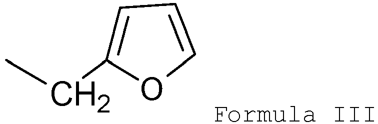
R³ is -H, -CH₃, -CH₂-CH₃ or -OCH₃,
R⁴ is -H or -CH₃,
R⁵ is -H or -CH₃ and
n is 1 or 2;
or

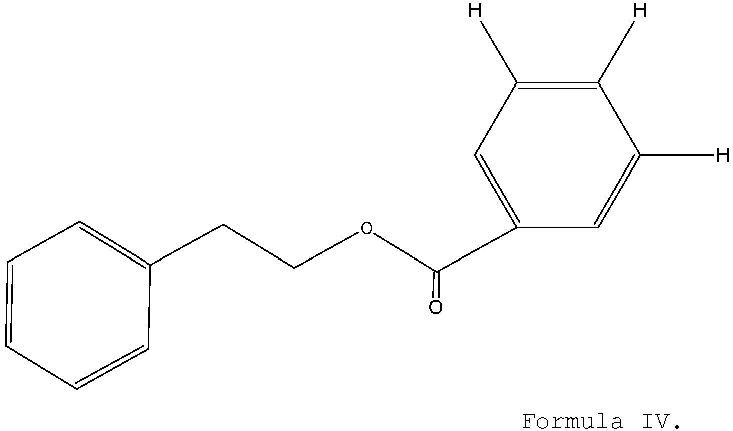

2. Method according to Claim 1, **characterized in that** the embedding medium contains 10% to 100% by volume of the aromatic ester and 0% to 90% by volume of an optically suitable, inert organic solvent having a refractive index of about 1.3, preferably 1.5.

3. Method according to Claim 1, **characterized in that** the embedding medium contains 10% to 100% by volume of the aromatic ester and 0% to 90% by volume of an optically suitable, inert organic solvent having a refractive index of about 2.0, preferably 1.65.

4. Method according to any of the preceding claims, **characterized in that** the embedding medium consists of an aromatic ester corresponding to formula I or formula IV.

5. Method according to any of the preceding claims, **characterized in that** the aromatic ester is benzyl 2-phenylacetate, 4-methoxybenzyl phenylacetate, phenethyl benzoate or 2-furylmethyl phenylacetate, preferably benzyl 2-phenylacetate.

6. Method according to any of the preceding claims, **characterized in that** the dehydration step a) involves the use of dehydrating compositions composed of aqueous alcohol, ketone or ether.

7. Method according to any of the preceding claims, **characterized in that** the tissue sample, before it is dehydrated in step a) and optically cleared in step b),
• is fixed and/or
• is fixed with formaldehyde and/or
• is washed and/or
• is washed with water and/or
• is incubated in an aqueous alkaline solution and/or
• is delipidated with a detergent solution and/or
• is delipidated with an organic solvent and/or
• is bleached with oxidizing reagents and/or
• is decolourized with amino alcohols.

8. Method according to any of the preceding claims, **characterized in that** the tissue sample, before it is dehydrated in step a) and optically cleared in step b), is fixed and that the fixative is preferably selected from crosslinking fixatives such as formaldehyde, glutaraldehyde, acrolein, carbodiimides, diethyl pyrocarbonate, bisimidoesters or glyoxal or mixtures thereof, and/or coagulant fixatives such as alcohols and other organic solvents, acids, potassium dichromate, lead nitrate, copper sulfate and mercuric chloride, and mixtures thereof.

9. Method according to any of the preceding claims, **characterized in that** the optically cleared tissue sample is in a further step examined under a microscope in order to obtain an image of the internal structure of the sample, the microscope being a light microscope.

10. Kit for preparing biological tissue samples for light microscopy, comprising:
- a dehydrating solvent for dehydrating the tissue sample and
- an embedding medium for clearing the dehydrated tissue sample by placing the sample in the embedding medium,
wherein the dehydrating solvent is selected from ethanol, tetrahydrofuran, isopropanol, tert-butanol, 2,2'-thiodiethanol, trichloroethanol or acetone and is present in the form of dehydrating compositions of aqueous solvent with increasing concentrations of solvent and
the embedding medium is an aromatic ester of the formula I or formula IV, where 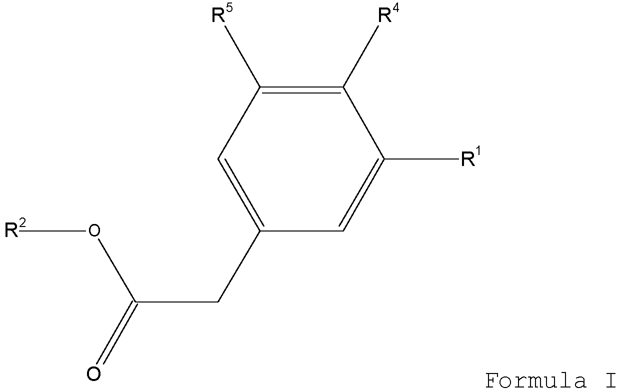 where
R¹ is -H, -CH₃ or -CH₂-CH₃,
R² is a radical of the formula II or formula III 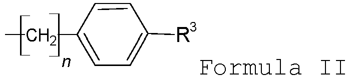 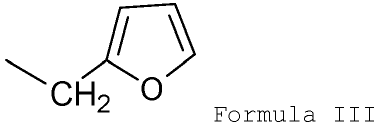
R³ is -H, -CH₃, -CH₂-CH₃ or OCH₃ ,
R⁴ is -H or -CH₃,
R⁵ is -H or -CH₃ and
n is 1 or 2;
or

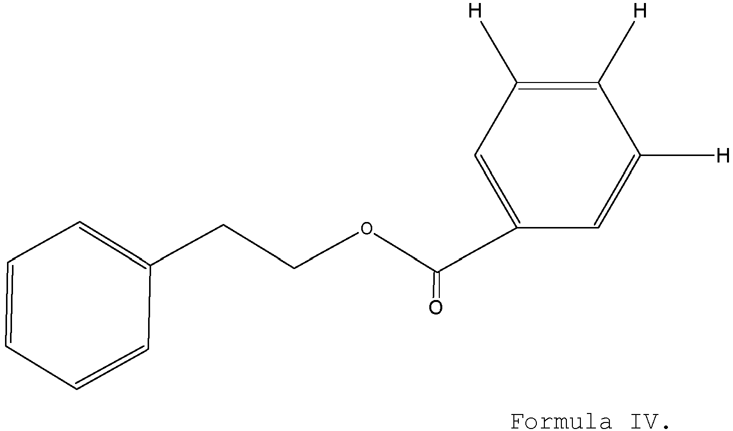

11. Kit according to Claim 10, **characterized in that** the aromatic ester is benzyl 2-phenylacetate, 4-methoxybenzyl phenylacetate, phenethyl benzoate or 2-furylmethyl phenylacetate, preferably benzyl 2-phenylacetate.

12. Use of an aromatic ester corresponding to formula I or corresponding to formula IV, where 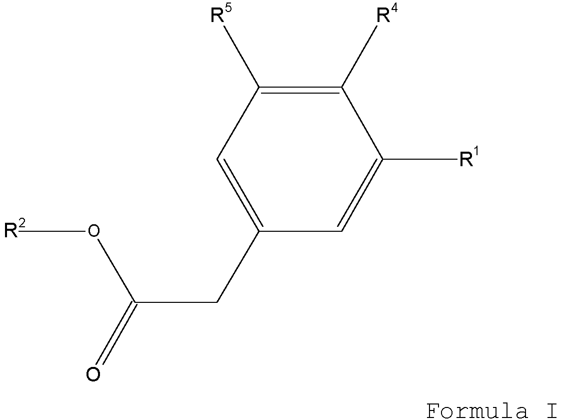 where
R¹ is -H, -CH₃ or -CH₂-CH₃,
R² is a radical of the formula II or formula III 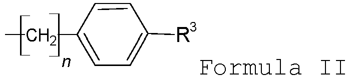 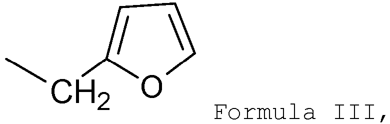
R³ is -H, -CH₃, -CH₂-CH₃ or OCH₃ ,
R⁴ is -H or -CH₃,
R⁵ is -H or -CH₃ and
n is 1 or 2; or 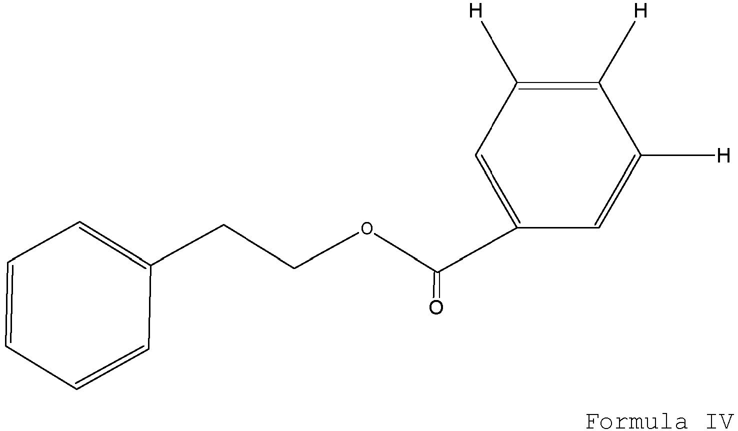
as an embedding medium for preparing a biological tissue sample for examination by light microscopy.

## Revendications

1. Procédé de fabrication d'échantillons de tissus transparents d'un tissu biologique pour examen au microscope optique, comprenant les étapes :
a) déshydratation de l'échantillon de tissu avec un solvant déshydratant et
b) clarification de l'échantillon de tissu déshydraté par introduction dans un milieu d'enrobement contenant un ester aromatique selon la formule I ou IV, avec 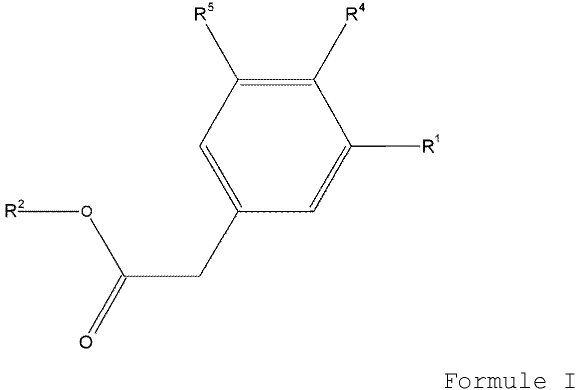 dans laquelle
R¹ = -H, -CH₃ ou -CH₂-CH₃,
R² représente un radical de formule II ou de formule III 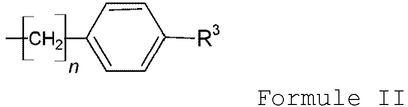 I 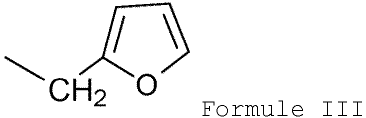
R³ = -H, -CH₃, -CH₂-CH₃ ou -OCH₃,
R⁴ = -H ou -CH₃
R⁵ = -H ou -CH₃ et
n = 1 ou 2 ;
ou

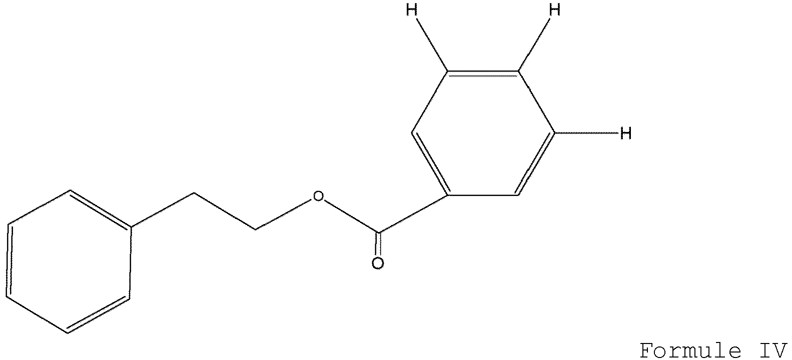

2. Procédé selon la revendication 1, **caractérisé en ce que** le milieu d'enrobement contient 10 à 100 % en volume de l'ester aromatique et contient 0 à 90 % en volume d'un solvant organique inerte optiquement approprié ayant un indice de réfraction d'environ 1,3, de préférence de 1,5.

3. Procédé selon la revendication 1, **caractérisé en ce que** le milieu d'enrobement contient 10 à 100 % en volume de l'ester aromatique et contient 0 à 90 % en volume d'un solvant organique inerte optiquement approprié ayant un indice de réfraction d'environ 2,0, de préférence de 1,65.

4. Procédé selon l'une des revendications précédentes, **caractérisé en ce que** le milieu d'enrobement est constitué d'un ester aromatique selon la formule I ou la formule IV.

5. Procédé selon l'une des revendications précédentes, **caractérisé en ce que** l'ester aromatique est le 2-phénylacétate de benzyle, le phénylacétate de 4-méthoxybenzyle, le benzoate de phénéthyle ou le phénylacétate de 2-furylméthyle, de préférence le 2-phénylacétate de benzyle.

6. Procédé selon l'une des revendications précédentes, **caractérisé en ce que** l'étape de déshydratation a) comprend l'utilisation de compositions déshydratantes constituées d'un alcool, d'une cétone ou d'un éther aqueux.

7. Procédé selon l'une des revendications précédentes, **caractérisé en ce que** l'échantillon de tissu, avant d'être déshydraté dans l'étape a) et optiquement clarifié dans l'étape b), est
• fixé et/ou
• fixé avec du formaldéhyde et/ou
• lavé et/ou
• lavé à l'eau et/ou
• incubé dans une solution alcaline aqueuse et/ou
• délipider avec une solution d'un détergent, et/ou
• délipider avec un solvant organique, et/ou
• blanchi avec des réactifs oxydants et/ou
• décoloré avec des amino-alcools.

8. Procédé selon l'une des revendications précédentes, **caractérisé en ce que** l'échantillon de tissu, avant d'être déshydraté dans l'étape a) et optiquement clarifié dans l'étape b), est fixé, et l'agent de fixation est choisi parmi les fixatifs réticulables tels que le formaldéhyde, le glutaraldéhyde, l'acroléine, le carbodiimide, le pyrocarbonate de diéthyle, les bisimodo-esters ou le glyoxal ou les mélanges de ceux-ci, et/ou les fixatifs coagulables tels que les alcools et d'autres solvants organiques, les acides, le bichromate de potassium, le nitrate de plomb, le sulfate de cuivre et le chlorure de mercure et les mélanges de ceux-ci.

9. Procédé selon l'une des revendications précédentes, **caractérisé en ce que** l'échantillon de tissu optiquement clarifié est, dans une autre étape, examiné au microscope, pour obtenir une image de la structure interne de l'échantillon, le microscope étant un microscope optique.

10. Trousse pour la préparation d'échantillons de tissus biologiques pour microscopie optique, contenant :
- un solvant déshydratant pour déshydrater l'échantillon de tissu et
- un milieu d'enrobement pour clarifier l'échantillon de tissu déshydraté par introduction dans le milieu d'enrobement,
le solvant déshydratant étant choisi parmi l'éthanol, le tétrahydrofurane, l'isopropanol, le tert-butanol, le 2,2'-thiodiéthanol, le trichloréthanol ou l'acétone ou sous forme de compositions déshydratantes d'un solvant aqueux présentant des concentrations croissantes du solvant, et
le milieu d'enrobement étant un ester aromatique de formule I ou de formule IV, avec 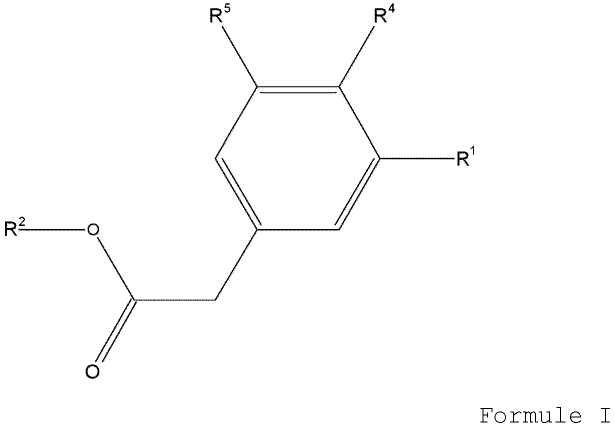 dans laquelle
R¹ = -H, -CH₃ ou -CH₂-CH₃,
R² représente un radical de formule II ou de formule III 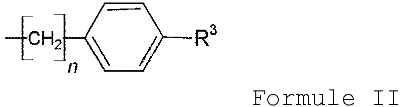
I 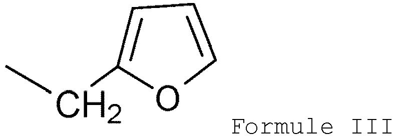
R³ = -H, -CH₃, -CH₂-CH₃ ou OCH₃,
R⁴ = -H ou -CH₃
R⁵ = -H ou -CH₃ et
n = 1 ou 2 ;
ou

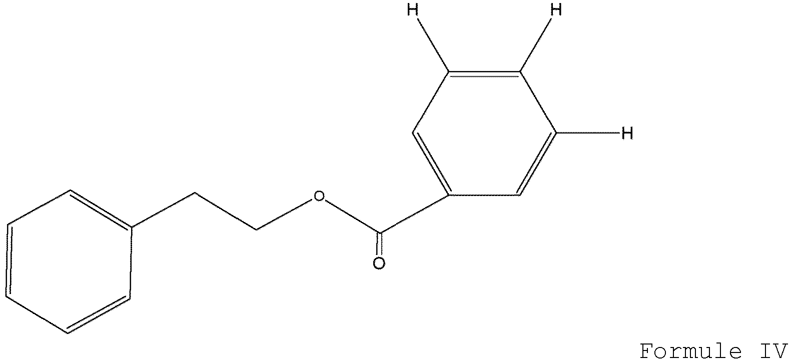

11. Trousse selon la revendication 10, **caractérisée en ce que** l'ester aromatique est le 2-phénylacétate de benzyle, le phénylacétate de 4-méthoxybenzyle, le benzoate de phénéthyle ou le phénylacétate de 2-furylméthyle, de préférence le 2-phénylacétate de benzyle.

12. Utilisation d'un ester aromatique selon la formule I ou la formule IV avec 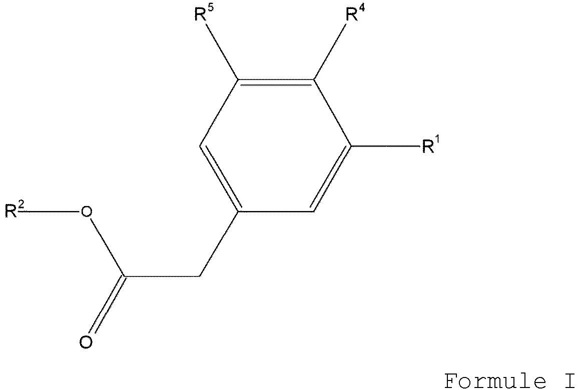 dans laquelle
R¹ = -H, -CH₃ ou -CH₂-CH₃,
R² représente un radical de formule II ou de formule III 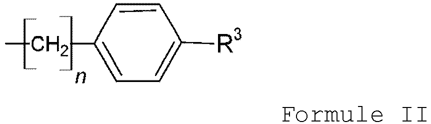
I 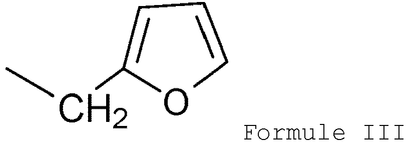 R³ = -H, -CH₃, -CH₂-CH₃ ou OCH₃,
R⁴ = -H ou -CH₃
R⁵ = -H ou -CH₃ et
n = 1 ou 2 ; ou 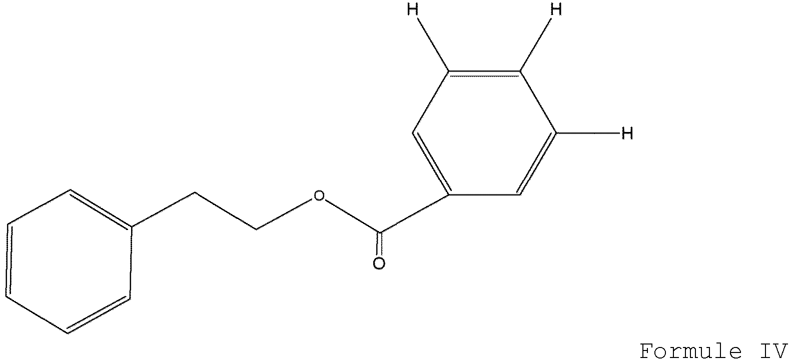
comme un milieu d'enrobement pour la préparation d'un échantillon biologique pour examen au microscope optique.
